Europäisches Patentamt

European Patent Office

Office européen des brevets

(11) Publication number: **0 447 984 A1**

# EUROPEAN PATENT APPLICATION

(21) Application number: **91104038.4**

(22) Date of filing: **15.03.91**

(51) Int. Cl.5: **A61K 39/42**, A61K 39/29, C07K 15/00

(30) Priority: **20.03.90 US 496489**

(43) Date of publication of application: **25.09.91 Bulletin 91/39**

(84) Designated Contracting States: **AT BE CH DE ES FR GB IT LI NL**

(71) Applicant: **ABBOTT LABORATORIES CHAD-0377, AP6D/2, One Abbott Park Road Abbott Park, Illinois 60064-3500(US)**

(72) Inventor: **Cummins, Laurence M. 314 Carter Avenue Libertyville, Illinois 60048(US)** Inventor: **Peterson, David A. 6134 218th Avenue Bristol, Wisconsin 53104(US)**

(74) Representative: **Modiano, Guido et al MODIANO, JOSIF, PISANTY & STAUB Modiano & Associati Via Meravigli, 16 I-20123 Milano(IT)**

(54) Hyperimmune globulin against hepatitis C virus and method for making same.

(57) A hyperimmune globulin composition of anti-Hepatitis C Virus (HCV) antibody, a hyperimmune globulin preparation against HCV having an antibody titer to HCV in the range of from about 1:2,000 to about 1:200,000 and a method for making both the composition and the preparation of the invention. In the inventive method, plasma from acceptable donors is pooled, fractionated, and rendered suitable for either intravenous or intramuscular injection. The preparation may be administered to individuals either exposed to HCV or at risk of HCV infection so that antibody titers to HCV may be increased in the serum of the individual.

EP 0 447 984 A1

## BACKGROUND OF THE INVENTION

### Field of the Invention

This invention relates generally to the Hepatitis C Virus (HCV), and more particularly, relates to a hyperimmune globulin having an antibody titer against HCV of sufficient potency to be an effective prophylactic against HCV, and a method for making the hyperimmune globulin.

### Related Art

The so-called non-A, non-B Hepatitis (NANBH) agent(s) account for approximately 90% of the cases of post-transfusion hepatitis reported in the United States. F. B. Hollinger et al. in N. R. Rose et al., eds., Manual of Clinical Immunology, American Society for Microbiology, Washington, D.C. (1986) 558-572. Chronic infection and hepatitis occur in approximately 50% of these cases; approximately 20% develop further to cirrhosis. J. L. Dienstag et al., Sem. Liv. Disease 6:67-81 (1986). The NANBH agent(s) also may be responsible for approximately 15-30% of the reported cases of sporadic hepatitis occurring in developed countries. Ibid. The chronicity of NANBH accounts for an increasing population of NANBH carriers.

The prevention of post-transfusion hepatitis is important not only to decrease the number of individuals suffering from chronic hepatitis but also to diminish the growing number of carriers of the agent(s). The prevention of NANBH theoretically can be accomplished by eliminating the agent(s) from the blood supply through effective screening procedures to detect its presence, active immunization of those individuals susceptible to the agent(s) with an effective and safe antigenic stimulus, passive immunization of an exposed or at risk individual with an antibody either in the form of whole serum or a fractionated, concentrated immune (gamma) globulin of predominantly immunoglobulin (Ig) G, or a combination of these and possibly other factors.

Immunoassays which detect some of the causative agents of post-transfusion hepatotropic infections such as Hepatitis B Virus (HBV), Cytomegalovirus (CMV), Hepatitis A Virus (HAV) and Epstein-Barr Virus (EBV), are available to screen potential blood transfusions for these agents, but no U.S. Government approved, commercially available immunoassay is available for the clinical detection of NANBH. This difficulty in detecting NANBH is due in part to the extraordinary problems encountered in correctly identifying antigens associated with NANBH. See, for example, J. Wands et al., U.S. Patent No. 4,870,076; J. Wands et al., Proc. Nat'l. Acad. Sci. USA 83:6608-6612 (1986); Ohori et al., J. Med. Virol. 12:161-176 (1983); Bradley et al., Proc. Nat'l. Acad. Sci. USA 84:6277-6287 (1987); T. Akatsuka et al., J. Med. Virol. 20:43-56 (1986); B. Seto et al., U.S. Patent Application Serial No. 07/234,641 (available from the U.S. Department of Commerce National Technical Information Service, Springfield, Virginia, No. 89138168); and R. Seelig et al., PCT International Application No. PCT/EP88/00123.

Recently, cDNA sequences of an RNA molecule were isolated from a cDNA library derived from plasma obtained from a chimpanzee with chronic liver disease. An immunoassay utilizing these recombinant sequences to detect the presence of this agent is described in published European Patent Application No. 0 318 216. These cDNA sequences encode antigens which react immunologically with antibodies present in individuals infected with NANBH. This agent, now termed the Hepatitis C Virus (HCV), thus has been established as a causative agent of NANBH. Choo et a., Science 244:359-362 (1989).

The immunization of susceptible individuals with a live or attenuated infectious agent is available as a preventive measure against various infectious agents including HBV, but no vaccine currently is available against HCV. The problems that hamper immunoassay development for HCV also impact the successful manufacture of a vaccine against HCV.

The administration of specific, high-titered gamma globulin to an individual who either cannot form antibodies, is at risk of infection or is exposed to an infectious agent can provide immediate protection to the individual when the gamma globulin is administered before or soon after exposure to the infectious agent. Passive immunization thus is useful for both immunocompromised and normal individuals. S. N. Cohen in D. P. Stites et al., Basic & Clinical Immunology, 6th Ed., Appleton & Lange, Norwalk, Connecticut (1987) 669-689.

Immune globulin (IG) therapy has been evaluated as a means of preventing HCV post-transfusion hepatitis. A. Sanchez-Quijano et al. report that only 3% of individuals receiving blood transfusions and undergoing cardiac surgery developed post-transfusion hepatitis when administered immune globulin both before and one week following transfusion and surgery. In contrast, 11% of the control individuals who received blood transfusions, underwent cardiac surgery and who were not administered immune globulin developed post-transfusion hepatitis. NANBH was diagnosed only when post-transfusion hepatitis infections due to HAV, HBV, CMV, EBV or herpes virus infections were ruled out serologically. A. Sanchez-Quijano et a., Lancet i:1245-1249 (1988).

The manufacture of an effective and specific high-titered immune globulin, termed hyperimmune globulin, against HCV is complicated by the need to provide a hyperimmune globulin preparation of sufficient potency to be effective against HCV which is also free of infectious agents, including the Human Immunodeficiency Virus (HIV) and HCV. The inactivation of infectious agents thus is important to the manufacture of hyperimmune globulin.

The gamma globulin fraction of plasma containing predominantly IgG may be obtained by methods known to those skilled in the art. These methods include polyethylene glycol fractionation as taught by Polson and Ruiz-Bravo, Vox Sang 23:107-118 (1972) and cold alcohol fractionation as taught in E. J. Cohn et al., J. Am. Chem. Soc. 68:459-475 (1946) and J. L. Oncley, J. Am. Chem. Soc. 71:541-550 (1949), which are incorporated herein by reference. In the cold alcohol method, a series of fractionation steps are performed in the presence of cold ethanol at a neutral pH between 6.8 and 7.3 in order to obtain the gamma globulin fraction of plasma which also is free of infectious agents. The fractionated immune globulin then can be treated further, if desired, with low levels of pepsin at pH 4 as taught by Reid et al., Vox Sang 55:75-80 (1988).

Methods currently employed to manufacture immune globulin do not always provide adequate inactivation of infectious agents. For example, patients administered immune globulin therapy prepared according to the pH 4/mild pepsin method reportedly developed HCV hepatitis as a result of the therapy. Williams et al., Vox Sang 57:15-18 (1989).

A need therefore exists for an improved hyperimmune globulin against HCV having an anti-HCV antibody titer of sufficient potency against HCV to be an effective prophylactic against HCV, while being substantially free of known infectious agents. A need further exists for a method for making such a hyperimmune globulin.

## SUMMARY OF THE INVENTION

This invention provides a hyperimmune globulin composition of anti-HCV antibody. This invention also provides a hyperimmune globulin preparation against HCV having an antibody titer to HCV in the range of from about 1:2,000 to about 1:200,000. The hyperimmune globulin preparation of the invention can be administered by either intravenous or intramuscular injection.

The present invention also provides a method of making both the hyperimmune globulin composition and the hyperimmune globulin preparation mentioned above. The method of the invention comprises the steps of (a) obtaining plasma from human donors which plasma has an antibody titer to HCV of at least about 1:500; (b) combining the plasma so obtained to form a pooled plasma; and (c) preparing either the composition or the preparation of the invention from the pooled plasma.

## DETAILED DESCRIPTION OF THE INVENTION

As previously stated, the present invention provides a hyperimmune globulin composition of anti-HCV antibody as well as a hyperimmune globulin preparation based upon such composition, having an antibody titer to HCV preferably in the range of about 1:2,000 to about 1:200,000. Alternatively, the range of the anti-HCV antibody titer in the preparation can be from about 1:2,000 to about 1:100,000 or from about 1:2,000 to about 1:50,000. The hyperimmune globulin preparation against HCV is provided such that an effective amount thereof can be administered intravenously or intramuscularly to an individual exposed to HCV or at risk to HCV, so that antibody titers to HCV are increased in the serum of the individual.

The practice of the method of the invention involves several steps. First, prospective donor plasma is screened to determine the anti-HCV antibody titer in such plasma. The plasma may be screened further to detect whether other infectious agents such as HIV and HBV are present. Next, pooled plasma is prepared by combining the donor plasma thus obtained, which has an acceptable antibody titer to HCV. The plasma is then fractionated, thereby obtaining that fraction of plasma comprising the gamma globulin fraction. Various methods of fractionating plasma such as the polyethylene glycol and cold alcohol fractionation methods described hereinabove can be employed. These steps also serve to substantially inactivate a majority of known infectious agents. If an intravenous preparation is desired, ion exchange chromatography can thereafter be employed to further purify the gamma globulin fraction. Each of the foregoing steps now will be described in greater detail.

In the practice of the present invention, hyperimmune globulin against HCV can be prepared from the plasma of human donors having an acceptable titer of antibody to HCV. Preferably, the donor plasma will contain an antibody titer to HCV of at least about 1:500. While human plasma useful in the present invention typically can be obtained by plasmapheresis from selected donors having acceptable antibody titer to HCV as previously described, other conventional methods also can be employed to obtain the donor plasma, including isolation from placental tissues or plasma from whole blood donations. A suitable donor may, for example, have an acceptable titer against HCV for purposes of the present invention resulting from previous exposure to HCV or from previous stimulation with a plasma protein product containing inactivated NANBH agent(s). Such stimulation can be achieved by injecting the donor with a suitable and effective amount of an inoculum. The inoculum, for example, can be a blood protein fraction, a viral or recombinant antigen or fragment thereof or a synthetic HCV peptide.

Prospective human donor plasma can be screened for antibody titer to HCV by known conventional methods, and thus the method used for such screening is not critical to the practice of the invention. For example, various immunoassays that involve the formation of antigen-antibody complexes and the detection of the formed complexes can be employed to determine the HCV antibody titer. Examples of known methods include enzyme-linked immunosorbent assays (ELISA) or other assays that employ an antibody "sandwich" assay technique which utilize enzymes, radiolabels or other detectable substances attached to the second antibodies. Competitive assays also can be employed, wherein antibody in the sample competes with labelled antibody for binding to HCV antigens. Various Hepatitis C antigens can be employed to determine the anti-HCV antibody titer of the donor, for example, the C100-3 protein described by Kuo et al., Science 244:362-364 (1989).

It is possible to obtain acceptably titered anti-HCV plasma by measuring the anti-HCV antibody titers in plasma of prospective donors regardless of their alanine aminotransferase (ALT) levels, or alternatively, acceptable donors may be selected by measuring the anti-HCV antibody titers in plasma of prospective donors who have elevated ALT levels.

Prospective donors identified as previously described can be screened for the presence or indication of other infectious agents, such as HIV antigen or antibody and HBV antigen. Methods to detect these antigens and antibodies are well-known to those of ordinary skill in the art. Donor plasma in which such antigens or antibodies are detected is not preferred for use in the practice of the present invention; it is preferable that plasma obtained from the prospective donors determined to be acceptable by the above criteria be combined to form the pooled plasma for use in the invention.

Other possible contaminating viruses, more specifically lipid coated viruses, can be inactivated in the pooled plasma by contacting the pooled plasma with a di- or tri-alkylphosphate. For example, a preferred inactivation technique involves using tri-(n-butyl) phosphate (TNBP), and a nonionic detergent such as polyoxyethylenesorbitan, commercially available under the name Tween 80™ from Fisher Scientific, Pittsburgh, PA, according to the method taught in U.S. Patent No. 4,540,573, which is incorporated herein by reference. In this method, a solution of 1% TNBP (wt/wt) and 1% Tween 80 (wt/wt) is added to the pooled plasma and the plasma is heated at 30° C ± 2° C for approximately four (4) hours.

In the method of the invention, the pooled plasma next is fractionated to obtain the gamma globulin fraction containing predominantly the IgG fraction. The preferred fractionation method is the Cohn-Oncley alcohol fractionation method, previously incorporated herein by reference and modified as herein described. This method involves a series of centrifugation steps in which the gamma globulin fraction is extracted from plasma by controlling alcohol concentration, pH and temperature.

The pH of the pooled plasma is adjusted to pH 7.2 by using a pH 4 acetate buffer (4.0 M acetic acid, 0.8 M sodium acetate) while maintaining the temperature of the pooled plasma at 1 to 5° C. Alcohol (a mixture of 95% ethanol and 5% methanol) is added to a final alcohol concentration of 8% in the plasma while the temperature of the plasma is lowered further to -1.5 to -3.0° C. A fraction precipitate forms, Fraction I, which consists predominantly of fibrinogen. The Fraction I precipitate is removed by centrifugation and then discarded, while the Fraction I supernatant is retained and further fractionated.

Alcohol is added to the Fraction I supernatant to achieve a 20% concentration of alcohol in the supernatant mixture. Next, the pH of the mixture is adjusted to pH 6.6 to 6.9 by adding the pH 4 acetate buffer previously described while the temperature of the mixture is lowered to -3 to -5° C. The mixture then is reacted for at least three hours, during which time a combined fraction (Fraction II and Fraction III) precipitate forms. This combined precipitate, which contains the gamma globulins as well as other plasma proteins, is removed by centrifugation.

The combined Fraction II and Fraction III precipitate is resuspended in 2.0 liters (L) of ice water (1.0 ± 1.0° C) per kilogram (kg) of precipitate. The precipitate is further dissolved in 2.0 L of 0.33 M sodium acetate solution (1 to 5° C) per kg of precipitate by adding the sodium acetate solution to the mixture. The pH of the mixture is adjusted to pH 5.2 ± 0.1 with the pH 4 acetate buffer previously described, and 17.2 L of ice water per kg of precipitate is added to the mixture. Alcohol then is added to a final alcohol concentration of 17% in the mixture. The temperature of the mixture then is lowered to -3.5 to -7.0° C. The Fraction III precipitate, which contains isoagglutinins, plasminogen and prothrombin, is removed by centrifugation and then discarded. The Fraction III supernatant then is clarified by filtration through depth filters.

The pH of the Fraction III filtrate next is adjusted to pH 6.8 or higher by using a 1 M sodium bicarbonate buffer. Alcohol then is added to obtain a final alcohol concentration of 25% while the temperature of the filtrate mixture is lowered to -4.5 to -7.5° C. The temperature of the mixture then is maintained between -5° C and -10° C. If necessary, the pH of the filtrate mixture is adjusted to pH 7.4 ± 0.2 by using a 1 M sodium bicarbonate buffer. This mixture is reacted for a minimum of six (6) hours, during which time the Fraction II precipitate which contains the gamma globulins forms. This Fraction II precipitate is removed by centrifugation.

Preferably, the hyperimmune globulin composition is prepared by employing both the TNBP/Tween 80 and the cold alcohol fractionation methodologies as described herein. However, residual TNBP/Tween 80, and/or some denatured IgG which occurred as a result of the chemical inactivation step, may be present in the hyperimmune globulin. It is preferred that an ion exchange step such as is described hereinbelow be performed to render a hyperimmune globulin free of denatured IgG and excess chemicals. This final ion exchange step yields an undenatured hyperimmune globulin against HCV which is most preferred for intravenous injection and also suitable for intramuscular injection. The final hyperimmune globulin composition is substantially pure monomeric IgG, that is, it contains at least about 95% monomeric IgG, most desirably, it contains 100% monomeric IgG, as determined by electrophoresis and HPLC methodologies.

Ion exchange chromatography using an anion exchanger is performed by methods known to those of ordinary skill in the art. Several factors influence the choice of a gel for the anion exchanger, including the pH of the eluting buffer and the buffer's salt concentration. See, for example, J. S. Baumstark et al., Arch. Biochem. Biophys. 108:514-522 (1964); and R. M. Condie, "Preparation and Intravenous Use of Undenatured Human IgG" in B. Alving and J. Finlayson, eds., Immunoglobulins: Characteristics and Uses of Intravenous Preparations (U.S. Government Printing Office, Washington, D.C., 1980).

The Fraction II precipitate, so-called Fraction II paste, is resuspended in 25 volumes of non-pyrogenic water and ice at 1.0 ± 1.0° C while mixing. Protein present in the Fraction II paste then is concentrated and buffer is exchanged by diafiltration. Macroscopic protein aggregates which formed during buffer exchange are removed by centrifugation. Monomeric IgG then is eluted by employing an ion exchange column containing diethyl[2-hydroxypropyl]aminoethyl as the functional group (known as a QAE-Sephadex® gel, available from Pharmacia, Piscataway, New Jersey), and imidazole buffer, pH 6.6. The collected effluent from the QAE column is replaced with 0.9% saline by diafiltration. After buffer exchange and concentration, the solution is centrifuged to remove macroscopic aggregates.

This hyperimmune globulin composition can be diluted to an approximate 5% protein solution with a pharmaceutically acceptable liquid carrier such as an isotonic solution of sodium chloride in water for injection to form a hyperimmune globulin preparation. Pharmaceutically acceptable preservatives also can be added to the hyperimmune globulin preparation in pharmaceutically acceptable amounts. Examples of these preservatives include glycine, maltose and thimerosal. Lastly, the hyperimmune globulin preparation can be sterilized by filtration. Optionally, the hyperimmune globulin preparation can be lyophilized.

The thus prepared hyperimmune globulin preparation against HCV can be administered in an effective amount by intravenous or intramuscular injection. It is known that administration of immune globulin by intravenous injection results in a more rapid increase of blood antibody titer. The route of administration and effective amount of the preparation depend on several factors including the physique of an individual, the physical condition of the individual, the stage of an individual's infection and the immunological state of the individual. Effective amounts of the preparation can be determined by the routineer.

Although the following specific examples illustrate several preferred embodiments of the present invention, they are not to be construed as limiting the scope of the invention in any manner.

EXAMPLE 1

Detection and Quantitation of Anti-HCV Antibody

An enzyme immunoassay (EIA) was employed to determine the antibody titer against HCV of prospective donors. Beads were coated with a recombinant antigen encoded by a nonstructural gene of HCV. The antigen was expressed as a fusion polypeptide containing 363 viral amino acids and human superoxide dismutase (C100-3), according to Kuo et al., Science 244:362-364 (1989). The coated beads were contacted with a donor sample diluted in normal human plasma and then incubated at 40 ± 2° C for approximately one (1) hour so that antigen-antibody complexes formed. Uncomplexed materials were aspirated and the beads were washed with distilled water. The beads then were contacted with goat anti-human IgG conjugated with horseradish peroxidase (HRPO), and complexes were allowed to form by incubating the beads and anti-human IgG at 40 ± 2° C for 30 ± 5 minutes. The beads then were washed with distilled water. A color signal was developed by adding o-phenylenediamine (OPD) solution containing hydrogen peroxide to the bead and incubating same for approximately 30 minutes at ambient room temperature in the dark. The reaction was stopped by adding 1 milliliter (mL) of 1 N $H_2SO_4$. The absorbance was read on a spectrophotometer at 492 nanometers (nm) ($A_{492}$). The intensity of the formed color signal was directly proportional to the amount of anti-HCV antibody present in the donor sample. The cutoff value was determined as 0.25 times the mean absorbance value of the positive control plus the mean absorbance value of the negative control. The anti-HCV antibody titer was determined to be the highest dilution at which the $A_{492}$ value of the sample was greater than or equal to the cutoff value.

EXAMPLE 2

Detection and Quantitation of Alanine Aminotransferase (ALT) Levels

The ALT levels of the prospective donors were tested according to the method of F. Wroblewski and J.S. LaDue, P. Soc. Exp. M. 91:569-71 (1956). The normal range for ALT activity was 0-75 International Units (IU)/L at the reference laboratories from which the prospective donor samples were received. An ALT level was considered to be elevated where it was greater than two times the upper limit of the normal range.

EXAMPLE 3

Selection of Donors

Plasma samples were obtained from 287 donors and then were assayed for anti-HCV antibody titer by the EIA method described in Example 1. Table 1 demonstrates the anti-HCV titer obtained from these donors in relationship to their ALT level as determined by the method of Example 2. As Table 1 demonstrates, 3 donors with normal ALT levels had anti-HCV antibody titers of 1:800 or greater, while 16 donors with elevated ALT levels exhibited anti-HCV antibody titers of 1:800 or greater.

## Table 1

### Anti-HCV Antibody Titers and ALT Levels of Prospective Donors

| ALT level | Number (N) of Donors* | Anti-HCV Antibody Titer of: | | | |
|---|---|---|---|---|---|
| | | 0 ** | 0-1:800 | 1:800-1:1600 | >1:1600 |
| Normal | 117 | 95 | 19 | 3 | 0 |
| Elevated | 170 | 77 | 77 | 10 | 6 |

*N=287; ** indicates no antibody was detected when undiluted donor sample was tested.

Stimulation of Donors for Anti-HCV Antibody

A suitable alternative source of anti-HCV plasma are healthy donors stimulated to produce anti-HCV antibody. Since no vaccine against HCV is currently available, blood protein fractions which contain inactivated HCV can be utilized for the stimulation of anti-HCV antibody production in such donors. Blood protein fractions suitable for stimulation include plasma protein fraction, Factor V, Factor VII, Factor VIII, Factor IX and fibrinogen. Methods of producing these fractions are known to those of ordinary skill in the art. A preferred blood protein is Factor VIII. Alternatively, inactivated viral or recombinant antigens of HCV or fragments thereof and synthetic HCV peptides may be used for stimulation.

EXAMPLE 4

Protective Efficacy of Hyperimmune Globulin Against HCV

The hyperimmune globulin preparation against HCV of the invention, formulated as previously described, can be tested for its protective efficacy against HCV as follows. A chimpanzee, serologically negative for anti-HCV antibody, is injected with the so-prepared hyperimmune globulin preparation in an amount equal to a concentration ranging from 50 to 500 milligrams (mg) of hyperimmune globulin per kg of body mass. One day following injection, the chimpanzee is challenged with an inoculum of 10 to 100 chimpanzee infectious doses (CID)/mL of Hutchinson strain NANBH which has been passaged in chimpanzees (the original human source of the inoculum is available from H. Alter, National Institutes of Health (NIH), Bethesda, Maryland). The lack of disease development demonstrates the pre-exposure prophylactic effect of the hyperimmune globulin since it has been documented by both elevated ALT levels and liver biopsies that such inoculum amounts result in NANBH in chimpanzees. Alter et al., Lancet i:459-63 (1978).

Other variations and modifications of the specific embodiments of the invention as set forth herein will be apparent to those skilled in the art. Accordingly, the invention is intended to be limited only in accordance with the appended claims.

**Claims**

1. A method for making a hyperimmune globulin composition against Hepatitis C Virus, which method comprises the steps of:
   (a) obtaining plasma from donors, said plasma having antibody titers to Hepatitis C Virus of at least about 1:500;
   (b) combining said plasma to form a pooled plasma; and
   (c) preparing a hyperimmune globulin composition against Hepatitis C virus from said pooled plasma.

2. A method for making a hyperimmune globulin composition against Hepatitis C Virus, which method comprises the steps of:
   (a) obtaining plasma from donors, said plasma having antibody titers to Hepatitis C of at least about 1:500;
   (b) combining said plasma to form a pooled plasma;
   (c) preparing a hyperimmune globulin composition against Hepatitis C from said pooled plasma of step (b) further comprising the steps of:
      (i) virally inactivating said pooled plasma; and
      (ii) fractioning said pooled plasma.

3. The hyperimmune globulin preparation according to the method of claim 1 or 2.

4. The method according to claim 2 wherein said hyperimmune globulin preparation is intravenously injectable.

5. A method for making a hyperimmune globulin preparation against Hepatitis C Virus which has an antibody titer to Hepatitis C Virus in the range of from about 1:2,000 to about 1:200,000, which method comprises the steps of:
   (a) obtaining plasma from donors, said plasma having antibody titers to Hepatitis C of at least about 1:500;
   (b) combining said plasma to form a pooled plasma;
   (c) preparing a hyperimmune globulin preparation against Hepatitis C from said pooled plasma of step (b) further comprising the steps of:
      (i) virally inactivating said pooled plasma; and
      (ii) fractionating said pooled plasma.

6. The method according to claim 5 wherein step (c) further comprises purifying said fractioned plasma by ion exchange chromatography and diluting said purified fraction to a final concentration of about 5% in the preparation.

7. The hyperimmune globulin preparation according to the method of claim 5 or 6.

8. The method according to claim 5 or 6 wherein said preparation is intravenously injectable.

9. A hyperimmune globulin composition of anti-Hepatitis C Virus antibody wherein said hyperimmune globulin comprises at least about ninety-five percent (95%) monomeric IgG.

10. A hyperimmune globulin preparation comprising an aqueous solution of a hyperimmune globulin composition of anti-Hepatitis C Virus antibody, said preparation having an antibody titer to Hepatitis C Virus in the range of from about 1:2,000 to about 1:200,000.

11. The preparation according to claim 10 which is intravenously injectable.

12. A method for stimulating antibody titer to Hepatitis C Virus in an individual, comprising injecting the individual with an effective amount of an inoculum selected from the group consisting of a blood protein fraction, a viral or recombinant antigen or fragment thereof and a synthetic HCV peptide.

European Patent Office

**PARTIAL EUROPEAN SEARCH REPORT**

which under Rule 45 of the European Patent Convention shall be considered, for the purposes of subsequent proceedings, as the European search report

Application number

EP 91104038

- page 1 -

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-61974 (C. TREPO) <br> * pages 26,27,30,31 * | 1-11 | A61K39/42 <br> A61K39/29 <br> C07K15/00 |
| X | WO-A-8912462 (GENELABS) <br> * pages 39-42; claim 22 * | 1-11 | |
| X,D | EP-A-318216 (CHIRON CORP.) <br> * claim 39 * | 1-11 | |
| X | FR-A-2609807 (INSTITUT PASTEUR) <br> * page 8 * | 1-11 | |
| X | WO-A-8200205 (BAXTER TRAVENOL LABORATORIES) <br> * page 12 * | 1-11 | TECHNICAL FIELDS SEARCHED (Int. Cl.5) |
| A | FR-A-2336141 (C. TREPO) <br> * pages 12,13 * | 1-11 | A61K <br> C07K |

...

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-11
Claims searched incompletely: —
Claims not searched: 12
Reason for the limitation of the search:

Article 52(4) EPC

| Place of search | Date of completion of the search | Examiner |
|---|---|---|
| Berlin | 23.04.1991 | P.F. AVEDIKIAN |

**European Patent Office**

## PARTIAL EUROPEAN SEARCH REPORT

| | DOCUMENTS CONSIDERED TO BE RELEVANT | | CLASSIFICATION OF THE APPLICATION (Int. Cl.5) |
|---|---|---|---|
| Category | Citation of document with indication, where appropriate, of relevant passages | Relevant to claim | |
| A | US-A-4346073 (D.L. ARONSON et al) <br> * the whole document * | 1-11 | |
| A,D | EP-A-279460 (R. SEELIG et al.) <br> * the whole document * | 1-11 | |
| | | | **TECHNICAL FIELDS SEARCHED (Int. Cl.5)** |

EPO Form 1505.3  06.78